# EUROPÄISCHE PATENTANMELDUNG

(11) **EP 1 553 059 A1**
(43) Veröffentlichungstag der Anmeldung: **13.07.2005**
(21) Anmeldenummer: 04450242.5
(22) Anmeldetag: 29.12.2004
(51) Int. Cl.: C02F 11/04, A23C 21/00

(54) **Verfahren und Vorrichtung zum Umwandeln und Verwerten von Nebenprodukten der milchverarbeitenden Industrie**

(30) Priorität: 09.01.2004 AT 200424
(71) Anmelder: Landfrisch Molkerei registrierte Genossenschaft mit beschränkter Haftung, 4600 Wels (AT); " Gradient" process technology GmbH, 1190 Wien (AT)
(72) Erfinder: Novalin, Senad, Dr., 1190 Wien (AT); Novalin-Canoy, Patricia, Mag., 1190 Wien (AT); Wöllinger, Johann, 4870 Vöcklamarkt (AT); Altendorfer, Herbert, Dir. Ing., 4132 Lembach (AT)
(74) Vertreter: Miksovsky, Alexander, Dipl.-Ing.

(57) **Zusammenfassung**

Bei einem Verfahren und einer Vorrichtung zum Umwandeln und Verwerten von Nebenprodukten der milchverarbeitenden Industrie, insbesondere von Molke, Molkepermeaten, Milchpermeaten oder dgl., sind folgende Schritte vorgesehen:
- Vergären (1) der Nebenprodukte, insbesondere von Molke (2), durch anaerobe Behandlung zur Erzeugung von Biogas (3);
- Verwerten des beim Vergären gewonnenen Biogases in einem Kraftwerk (4) unter Erzeugung von elektrischer und/oder thermischer Energie;
wobei vor dem Vergären (1) eine Reduktion von Feststoffen bzw. gelösten Stoffen, enthaltend insbesondere Calcium, Magnesium und Phosphat, vorgenommen wird und Inhaltsstoffe der Nebenprodukte, insbesondere Proteine, entfernt werden.

Dadurch gelingt eine einfache und ökonomisch sinnvoll durchführbare Umwandlung bzw. Verwertung von Nebenprodukten der milchverarbeitenden Industrie, insbesondere von Molke.

## Beschreibung

Die vorliegende Erfindung bezieht sich auf ein Verfahren sowie eine Vorrichtung zum Umwandeln und Verwerten von Nebenprodukten der milchverarbeitenden Industrie, insbesondere von Molke, Molkepermeaten, Milchpermeaten oder dgl.

Im Zusammenhang mit der Verwertung von Nebenprodukten der milchverarbeitenden Industrie, insbesondere von Molke und ähnlichen Produkten, entstehen teilweise ernsthafte Verwertungs- und Entsorgungsprobleme, da beispielsweise Molke in großen Mengen anfällt und einer Verwertung oder Entsorgung zuzuführen ist. Allgemein bezieht sich die vorliegende Erfindung auf die Umwandlung und Verwertung von Nebenprodukten der milchverarbeitenden Industrie, welche Laktose beinhalten, wenn auch im Folgenden primär auf eine Verwertung von Molke eingegangen wird.

Im Zusammenhang mit einer Verwertung bzw. Entsorgung von Molke ist derzeit davon auszugehen, daß ein großer Anteil beispielsweise in Form von Tierfutter, Dünger oder dgl. entsorgt wird.

Weiters ist bekannt, daß beispielsweise Inhaltsstoffe, insbesondere Proteine und Laktose, von Molke und ähnlichen Produkten potentielle Wirtschaftsgüter darstellen, so daß beispielsweise für aus Molke abzutrennende Laktose wirtschaftlich sinnvolle Verwertungsmöglichkeiten bestehen. Es ist beispielsweise bekannt, Laktose gegebenenfalls unter Einsatz von beispielsweise Biokatalysatoren oder Mikroorganismen zu anderen Produkten, wie beispielsweise Alkohol, Milchsäure oder Tagatose, umzuwandeln bzw. zu konvertieren.

Diese Verwertungsmöglichkeiten sind jedoch nur für vergleichsweise geringe Mengen von anfallender Molke sinnvoll einsetzbar.

Die vorliegende Erfindung zielt daher darauf ab, ein Verfahren und eine Vorrichtung zum Umwandeln und Verwerten von Nebenprodukten der milchverarbeitenden Industrie, insbesondere von Molke, Molkepermeaten, Milchpermeaten oder dgl., zur Verfügung zu stellen, mit welchen eine ökonomisch sinnvolle und durchführbare Umwandlung und Verwertung von Molke oder ähnlichen Nebenprodukten der milchverarbeitenden Industrie möglich wird, so daß beispielsweise bestehende Probleme im Hinblick auf eine Entsorgung von großen Mengen derartiger Nebenprodukte der milchverarbeitenden Industrie vermieden werden können.

Zur Lösung dieser Aufgaben umfaßt das Verfahren zum Umwandeln und Verwerten von Nebenprodukten der milchverarbeitenden Industrie, insbesondere von Molke, Molkepermeaten, Milchpermeaten oder dgl., im wesentlichen die folgenden Schritte:
- Vergären der Nebenprodukte, insbesondere von Molke, durch anaerobe Behandlung zur Erzeugung von Biogas;
- Verwerten des beim Vergären gewonnenen Biogases in einem Kraftwerk unter Erzeugung von elektrischer und/oder thermischer Energie;
wobei vor dem Vergären eine Reduktion von Feststoffen bzw. gelösten Stoffen, enthaltend insbesondere Calcium, Magnesium und Phosphat, vorgenommen wird und Inhaltsstoffe der Nebenprodukte, insbesondere Proteine, entfernt werden.

Durch das erfindungsgemäß vorgeschlagene Vergären von Nebenprodukten der milchverarbeitenden Industrie, insbesondere von Molke, durch anaerobe Behandlung zur Erzeugung von Biogas wird eine Umwandlung bzw. Konvertierung von Molke bzw. allgemein von Nebenprodukten der milchverarbeitenden Industrie in ein wirtschaftlich verwendbares bzw. verwertbares Produkt in Form von Biogas möglich, welches in weiterer Folge in einem gegebenenfalls an sich bekannten Kraftwerk, beispielsweise Blockheizkraftwerk, unter Erzeugung elektrischer und/oder thermischer Energie verwertet, insbesondere verbrannt, wird. Es läßt sich somit die in Nebenprodukten der milchverarbeitenden Industrie, insbesondere in Molke, enthaltene Energie nach bzw. durch Umwandlung in Biogas ökonomisch sinnvoll nutzen, wobei die aus dem Biogas gewonnene, elektrische und/oder thermische Energie beispielsweise zur wenigstens teilweisen Energieabdeckung des Industriebetriebs herangezogen werden kann, in welchem die Nebenprodukte anfallen bzw. angefallen sind. Derartige Feststoffe bzw. gelöste Stoffe, enthaltend insbesondere Calcium, Magnesium und Phosphat, können zu Problemen im Zusammenhang mit der Durchführung des Gärprozesses führen, so daß die Reduktion bzw. Entfernung derartiger Inhaltsstoffe gegebenenfalls unter einen vorbestimmten bzw. vorbestimmbaren Schwellwert vor dem Gärvorgang erfindungsgemäß vorgenommen wird. Durch eine Entfernung von Inhaltsstoffen, wie Proteinen, können diese einer entsprechenden Verwertung zugeführt werden.

Um den Vergärungsprozeß nicht durch eine gegebenenfalls vorhandene, große Substratbelastung zu beeinträchtigen, wird gemäß einer bevorzugten Ausführungsform so vorgegangen, daß vor dem Vergären ein Verdünnen der Nebenprodukte, insbesondere von Molke, insbesondere durch Zusatz von Wasser bzw. Abwasser vorgenommen wird. Ein derartiges Zusetzen von Wasser bringt eine Verdünnung der der Vergärung zuzuführenden Einsatzstoffe und bei gegebenenfalls vorhandener, hoher Substratbelastung eine entsprechende Verringerung der Schadstoffe, so daß der anaerobe Gärvorgang ohne Beeinträchtigung durchgeführt werden kann.

Für einen besonders günstigen und raschen Gärvorgang wird gemäß einer weiters bevorzugten Ausführungsform vorgeschlagen, daß das Vergären bei einer Temperatur von 20 bis 60 °C, insbesondere einer gegenüber Umgebungstemperatur erhöhten Temperatur, besonders bevorzugt bei etwa 35 bis 40 °C, vorgenommen wird.

Neben der oben erwähnten, bevorzugten Verdünnung der der Vergärung zuzuführenden Produkte, beispielsweise zur Reduktion der Substratkonzentration, wird gemäß einer weiters bevorzugten Ausführungsform vorgeschlagen, daß die Reduktion von Feststoffen bzw. gelösten Stoffen durch Erhöhen des pH-Werts des einem Vergären zuzuführenden Materials und/oder durch Ionenaustausch, Elektrolyse, Zentrifugation oder dgl. vorgenommen wird. Eine besonders einfache und zuverlässige Möglichkeit einer Reduktion bzw. einer Umwandlung derartiger Inhaltsstoffe der zu verarbeitenden bzw. zu verwertenden Nebenprodukte gelingt hiebei durch Erhöhen des pH-Werts beispielsweise mittels Zusatz von Lauge, insbesondere Natronlauge, wie dies gemäß einer weiters bevorzugten Ausführungsform erfindungsgemäß vorgeschlagen wird.

Wie oben bereits angedeutet, sind in Nebenprodukten der milchverarbeitenden Industrie darüber hinaus oftmals Inhaltsstoffe, wie beispielsweise Proteine und ähnliche, verwertbare Wirtschaftsgüter, enthalten, so daß gemäß einer weiters bevorzugten Ausführungsform vorgeschlagen wird, daß die Inhaltsstoffe der Nebenprodukte durch eine Mikrofiltration, Ultrafiltration, offenporige Nanofiltration oder präparative Chromatografie, entfernt werden. Es können somit derartige, in den zu verwertenden bzw. umzuwandelnden Nebenprodukten der milchverarbeitenden Industrie enthaltene Inhaltsstoffe vor einem Vergären abgetrennt und einer gesonderten Verwertung zugeführt werden.

Bei einer derartigen Abtrennung von Inhaltsstoffen, wie beispielsweise Proteinen, ist in manchen Fällen auch eine zu hohe Herabsetzung der für eine ordnungsgemäße Durchführung des Gärvorgangs notwendigen Stickstoffanteile zu beobachten, so daß gemäß einer weiters bevorzugten Ausführungsform vorgeschlagen wird, daß vor dem Vergären eine Stickstoffquelle, beispielsweise Harnstoff, den zu vergärenden Nebenprodukten zugesetzt wird. Durch einen derartigen Zusatz einer Stickstoffquelle, beispielsweise Harnstoff, können somit die für einen optimalen Gärprozeß erforderlichen Parameter beeinflußt bzw. eingestellt werden.

Um nach dem Vergären eine Verwertung, insbesondere problemlose Verbrennung, des Biogases in einem Kraftwerk vorzunehmen, wird gemäß einer weiters bevorzugten Ausführungsform vorgeschlagen, daß nach dem Vergären eine Reinigung, insbesondere eine Entschwefelung des Biogases durchgeführt wird. Zur weiteren Reduktion einer Schadstoffbelastung des Biogases vor der Verbrennung wird gemäß einer weiters bevorzugten Ausführungsform vorgeschlagen, daß nach dem Vergären eine weitere Reinigung, insbesondere mittels Adsorption des Biogases durchgeführt wird.

Wie bereits oben angedeutet, kann die beispielsweise unter Verwertung des beim Vergären gewonnenen Biogases in einem Kraftwerk erzeugte Energie in Form von elektrischer und/oder thermischer Energie beispielsweise unmittelbar in dem Industriebetrieb herangezogen werden, in welchem die zu verwertenden Nebenprodukte anfallen. Im Zusammenhang mit einer milchverarbeitenden Industrie ist eine Vielzahl von unterschiedlichen Verfahrensschritten bzw. Verfahren einer Verwertung von Milchprodukten bekannt, wobei vorgeschlagen wird, daß die im Kraftwerk gewonnene, thermische Energie zur Erzeugung von Wärme und/oder Kälte herangezogen wird, wie dies einer weiters bevorzugten Ausführungsform des erfindungsgemäßen Verfahrens entspricht. Es kann somit abgestimmt auf die im Rahmen des Industriebetriebs herzustellenden Produkte eine gezielte Verwertung insbesondere der thermischen Energie im Hinblick auf einen Einsatz in Form von Wärme und/oder Kälte, beispielsweise zum Wärmebehandeln von Milchprodukten und/oder Kühlen derselben, eingesetzt werden. Hiebei zeigt sich, daß insbesondere durch Einsetzen der aus dem Biogas gewonnenen Energie in Form von sowohl elektrischer als auch thermischer Energie der Wirkungsgrad bzw. Gesamtenergiewirkungsgrad eines erfindungsgemäß vorgesehenen Kraftwerks auf über 75 %, insbesondere über 85 %, gesteigert werden kann.

Zur Lösung der eingangs genannten Aufgaben umfaßt eine Vorrichtung zum Umwandeln und Verwerten von Nebenprodukten der milchverarbeitenden Industrie, insbesondere von Molke, Molkepermeaten, Milchpermeaten oder dgl., im wesentlichen:
eine Einrichtung zum Vergären der Nebenprodukte, insbesondere von Molke, durch anaerobe Behandlung zur Erzeugung von Biogas;
ein Kraftwerk zum Erzeugen von elektrischer und/oder thermischer Energie aus dem beim Vergären gewonnenen Biogas; und
eine Einrichtung zum Abtrennen von Inhaltsstoffen, insbesondere zum Abtrennen von Calcium, Magnesium und Phosphaten und/oder von Proteinen, aus den zu vergärenden Nebenprodukten.

Wie oben bereits ausführlich erörtert, gelingt somit mit konstruktiv einfachen Mitteln eine zuverlässige Umwandlung und Verwertung von in der milchverarbeitenden Industrie anfallenden Nebenprodukten, insbesondere von Molke, Molkepermeaten, Milchpermeaten oder dgl., durch Einsatz einer Einrichtung zum Vergären, beispielsweise eines Bioreaktors, und eines nachgeschalteten Kraftwerks, insbesondere Blockheizkraftwerks, zum Erzeugen von elektrischer und/oder thermischer Energie. Zur Entfernung von den Gärprozeß beeinträchtigenden Inhaltsstoffen und/oder zur Erhöhung der gesamten Wertschöpfung der zu verarbeitenden bzw. verwertenden Produkte wird erfindungsgemäß vorgeschlagen, daß zusätzlich eine Einrichtung zum Abtrennen von Inhaltsstoffen, insbesondere zum Abtrennen von Calcium, Magnesium und Phosphaten und/oder von Proteinen, aus den zu vergärenden Nebenprodukten vorgesehen ist.

Zur Einstellung von einen optimalen Gärprozeß ermöglichenden Parametern wird darüber hinaus vorgeschlagen, daß eine Einrichtung zum Zudosieren von Wasser bzw. Abwasser und/oder einer Stickstoffquelle, insbesondere von Harnstoff, vorgesehen ist, wie dies einer weiters bevorzugten Ausführungsform der erfindungsgemäßen Vorrichtung entspricht.

Zur Erzielung optimaler Gärparameter ist darüber hinaus bevorzugt vorgesehen, daß eine Einrichtung zum Erwärmen der zu vergärenden Nebenprodukte vorgesehen ist, wobei durch ein gezieltes Erwärmen bzw. Aufrechterhalten einer für den Gärprozeß optimalen Temperatur eine entsprechend rasche Umsetzung auch von gegebenenfalls großen Mengen anfallender Nebenprodukte, insbesondere von Molke möglich wird.

Um bei der nachfolgenden Verwertung des beim Vergären anfallenden Biogases eine problemlose Verwertung, insbesondere Verbrennung, von Biogas vornehmen zu können, wird gemäß einer weiters bevorzugten Ausführungsform vorgeschlagen, daß eine Vorrichtung zum Reinigen des Biogases, insbesondere eine Vorrichtung zum Entschwefeln des Biogases, zwischen der Einrichtung zum Vergären und dem Kraftwerk vorgesehen ist.

Um gegebenenfalls mögliche Schwankungen im Zusammenhang mit dem Anfall von erzeugtem Biogas und/oder dem Verbrauch an im nachgeschalteten Kraftwerk erzeugter, elektrischer und/oder thermischer Energie handhaben zu können, wird gemäß einer weiters bevorzugten Ausführungsform vorgeschlagen, daß zwischen der Einrichtung zum Vergären der Nebenprodukte und dem Kraftwerk eine Speichervorrichtung zum Speichern von erzeugtem Biogas vorgesehen ist.

Für eine Pufferung der zu verarbeitenden Nebenprodukte und/oder eine gegebenenfalls erforderliche Vorbehandlung ist gemäß einer weiters bevorzugten Ausführungsform vorgesehen, daß der Einrichtung zum Vergären der Nebenprodukte ein Puffer bzw. Vorratstank und/oder eine Konditionierungseinrichtung vorgeschaltet ist.

Die Erfindung wird nachfolgend anhand von in der beiliegenden Zeichnung schematisch dargestellten Ausführungsbeispielen näher erläutert. In dieser zeigen:
Fig. 1 eine schematische Darstellung einer ersten Ausführungsform einer erfindungsgemäßen Vorrichtung zur Durchführung des erfindungsgemäßen Verfahrens; und
Fig. 2 in ebenfalls schematischer Darstellung eine abgewandelte Ausführungsform einer erfindungsgemäßen Vorrichtung zur Durchführung des erfindungsgemäßen Verfahrens.

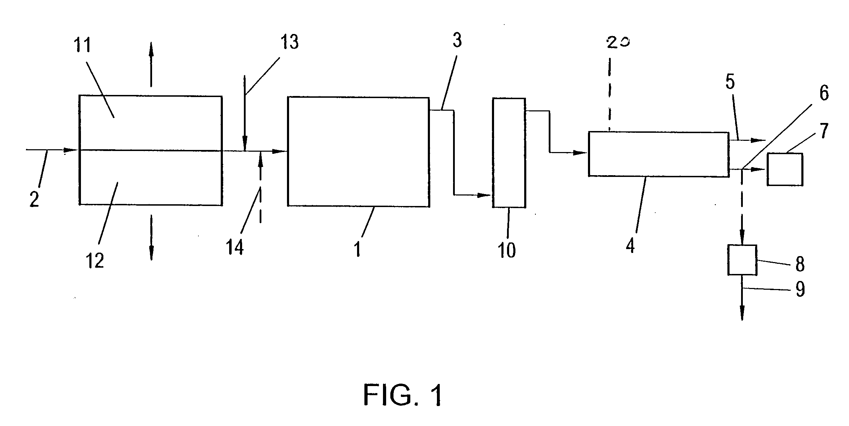

In Fig. 1 ist mit 1 eine Einrichtung zum Vergären von gemäß einem Pfeil 2 zuzuführenden Nebenprodukten der milchverarbeitenden Industrie, insbesondere von Molke, Molkepermeaten, Milchpermeaten oder dgl., angedeutet, wobei in der Einrichtung zum Vergären, welche beispielsweise von einem Bioreaktor 1 gebildet wird, ein anaerobes Vergären der Nebenprodukte, insbesondere von Molke, zur Erzeugung von Biogas erfolgt.

Das aus der Einrichtung zum Vergären bzw. dem Bioreaktor 1 abgezogene Abgas 3 wird in weiterer Folge in einem Kraftwerk, beispielsweise einem Blockheizkraftwerk 4, unter Erzeugung von elektrischer und/oder thermischer Energie verwertet bzw. verbrannt, wobei die Entnahme von elektrischer Energie mit 5 angedeutet ist und die Entnahme von thermischer Energie mit 6 angedeutet ist. Die bei 6 abgezogene, thermische Energie kann beispielsweise in Form von Wärme bzw. Hitze beispielsweise in einem Wärmetauscher 7 genutzt werden. Alternativ oder zusätzlich kann die bei 6 abgezogene, thermische Energie beispielsweise in einer Adsorptionskälteanlage 8 eingesetzt werden, aus welcher Kälte abgezogen werden kann, wie dies durch 9 angedeutet ist.

Um bei gegebenenfalls schwankenden Mengen des abgezogenen Abgases 3 einen Betrieb des Kraftwerks 4 in einem optimierten Betriebszustand aufrecht zu erhalten, ist eine Zuführung 20 zum Zuführen eines zusätzlichen Brennstoffes, beispielsweise Erdgas, strichliert angedeutet.

Für ein Reinigen des im Bioreaktor bzw. der Einrichtung 1 gewonnenen Biogases, welches bei 3 abgezogen wird, ist in Fig. 1 schematisch eine Reinigungsvorrichtung 10 insbesondere zum Entschwefeln des Biogases angedeutet. Zusätzlich bzw. nachgeschaltet oder alternativ könnte in der Einrichtung 10 auch eine Adsorption vorgenommen werden.

In Fig. 1 ist weiters angedeutet, daß vor einem unmittelbaren Einbringen der zu verwertenden Nebenprodukte, welche gemäß dem Pfeil 2 zugeführt werden, insbesondere zur Einstellung von einen optimalen Gärprozeß ermöglichenden Parametern, in einer Einrichtung 11 eine Abtrennung von verwertbaren Inhaltsstoffen, beispielsweise von Protein erfolgt, während in einer zusätzlichen Einrichtung bzw. Stufe 12 eine Abtrennung von anorganischen Inhaltsstoffen, wie beispielsweise Calcium, Magnesium, Phosphat usw., erfolgt.

Für eine weitere Optimierung des Gärprozesses ist in Fig. 1 darüber hinaus eine Zugabe von Wasser, beispielsweise Abwasser 13, angedeutet, um gegebenenfalls unverändert in der beispielsweise zu verarbeitenden Molke enthaltene Inhaltsstoffe weiter zu verdünnen. Weiters ist mit 14 eine Zugabe einer Stickstoffquelle, beispielsweise von Harnstoff, ebenfalls zur Optimierung des Gärprozesses angedeutet.

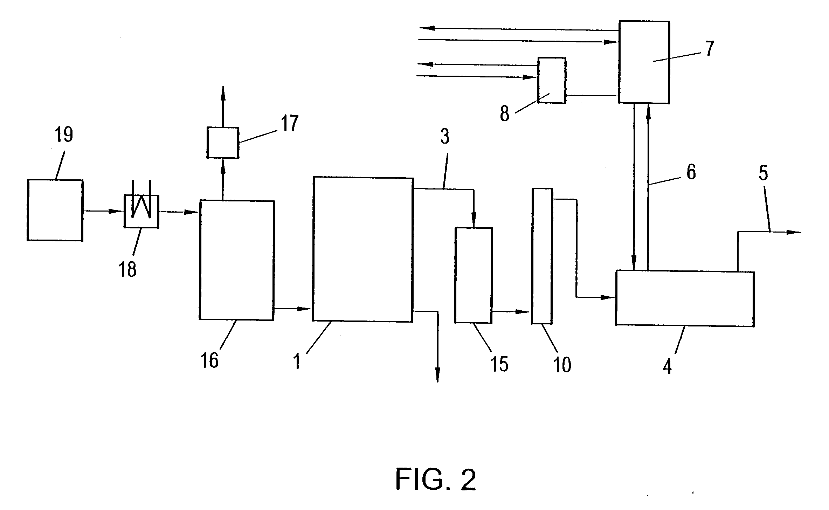

Bei der in Fig. 2 dargestellten, abgewandelten Ausführungsform erfolgt wiederum in einer Einrichtung bzw. einem Bioreaktor 1 ein Vergären von Nebenprodukten der milchverarbeitenden Industrie, wobei das wiederum bei 3 abgezogene Biogas in einem Kraftwerk, beispielsweise einem Blockheizkraftwerk 4, weiterverarbeitet bzw. verwertet wird.

Ähnlich wie bei der Ausführungsform gemäß Fig. 1 erfolgt bei 5 eine Entnahme bzw. ein Abziehen von elektrischer Energie, während bei 6 eine Entnahme thermischer Energie wiederum beispielsweise in einen mit 7 bezeichneten Wärmetauscher erfolgt, wobei zusätzlich wie bei der Ausführungsform gemäß Fig. 1 eine Kälteanlage mit 8 angedeutet ist. Alternativ könnte auch ein Wärmespeicher vorgesehen sein.

Zusätzlich zu der auch bereits bei der Ausführungsform gemäß Fig. 1 vorgesehenen Reinigungsvorrichtung 10 für das Biogas, beispielsweise zur Durchführung einer Entschwefelung, ist bei der Ausführungsform gemäß Fig. 2 ein Biogasspeicher 15 angedeutet, um einen gegebenenfalls unterschiedlichen Anfall von erzeugtem Biogas und/oder gegebenenfalls unterschiedlichen Verbrauch desselben in dem nachgeschalteten Kraftwerk 4 handhaben zu können.

Ähnlich wie bei der Ausführungsform gemäß Fig. 1 ist auch gemäß Fig. 2 der Einrichtung bzw. dem Bioreaktor 1 vorgeschaltet eine Konditionierungseinrichtung 16, in welcher zur Einstellung optimaler Gärparameter beispielsweise wiederum eine Entnahme von Inhaltsstoffen, wie beispielsweise Protein, eine Entfernung von anorganischen Inhaltsstoffen, insbesondere von Calcium, Magnesium und Phosphat, wie dies in Fig. 1 mit 11 und 12 angedeutet ist, als auch gegebenenfalls zusätzlich ein Zusatz beispielsweise von Wasser und/oder einer Stickstoffquelle vorgenommen wird. Dem Konditionierungsbehälter bzw. der Konditionierungseinrichtung 16 ist darüber hinaus beispielsweise ein Biofilter 17 nachgeschaltet.

Zur Erzielung bzw. Einhaltung von optimalen Temperaturbedingungen für den nachfolgenden Gärprozeß ist darüber hinaus in Fig. 2 ein Wärmetauscher 18 bzw. allgemein eine Einrichtung zum Erwärmen der aus einem Vorratstank 19 zu entnehmenden Molke vorgesehen. Anstelle des Wärmetauschers bzw. der Einrichtung 18 zum Erwärmen der zu verwertenden Nebenprodukte, insbesondere von Molke, kann eine derartige Heizeinrichtung beispielsweise auch unmittelbar im Inneren der Einrichtung bzw. des Bioreaktors 1 vorgesehen sein, um die für einen Gärprozeß optimalen Bedingungen einzustellen bzw. aufrecht zu erhalten.

## Patentansprüche

1. Verfahren zum Umwandeln und Verwerten von Nebenprodukten der milchverarbeitenden Industrie, insbesondere von Molke, Molkepermeaten, Milchpermeaten oder dgl., umfassend die folgenden Schritte:
- Vergären (1) der Nebenprodukte, insbesondere von Molke (2), durch anaerobe Behandlung zur Erzeugung von Biogas (3) ;
- Verwerten des beim Vergären gewonnenen Biogases in einem (4)Kraftwerk unter Erzeugung von elektrischer und/oder thermischer Energie;
wobei vor dem Vergären (1) eine Reduktion von Feststoffen bzw. gelösten Stoffen, enthaltend insbesondere Calcium, Magnesium und Phosphat, vorgenommen wird und Inhaltsstoffe der Nebenprodukte, insbesondere Proteine, entfernt werden.

2. Verfahren nach Anspruch 1, **dadurch gekennzeichnet, daß** vor dem Vergären ein Verdünnen der Nebenprodukte, insbesondere von Molke (2), insbesondere durch Zusatz von Wasser bzw. Abwasser vorgenommen wird.

3. Verfahren nach Anspruch 1 oder 2, **dadurch gekennzeichnet, daß** das Vergären (1) bei einer Temperatur von 20 bis 60 °C, insbesondere einer gegenüber Umgebungstemperatur erhöhten Temperatur, besonders bevorzugt bei etwa 35 bis 40 °C, vorgenommen wird.

4. Verfahren nach Anspruch 1, 2 oder 3, **dadurch gekennzeichnet, daß** die Reduktion von Feststoffen bzw. gelösten Stoffen durch Erhöhen des pH-Werts des einem Vergären zuzuführenden Materials und/oder durch Ionenaustausch, Elektrolyse, Zentrifugation oder dgl. vorgenommen wird.

5. Verfahren nach Anspruch 4, **dadurch gekennzeichnet, daß** das Erhöhen des pH-Werts durch Zugabe von Lauge, insbesondere Natronlauge, vorgenommen wird.

6. Verfahren nach einem der Ansprüche 1 bis 5, **dadurch gekennzeichnet, daß** die Inhaltsstoffe der Nebenprodukte durch eine Mikrofiltration, Ultrafiltration, offenporige Nanofiltration oder präparative Chromatografie entfernt werden.

7. Verfahren nach einem der Ansprüche 1 bis 6, **dadurch gekennzeichnet, daß** vor dem Vergären (1) eine Stickstoffquelle, beispielsweise Harnstoff, den zu vergärenden Nebenprodukten zugesetzt wird.

8. Verfahren nach einem der Ansprüche 1 bis 7, **dadurch gekennzeichnet, daß** nach dem Vergären (1) eine Reinigung, insbesondere eine Entschwefelung des Biogases durchgeführt wird.

9. Verfahren nach Anspruch 8, **dadurch gekennzeichnet, daß** nach dem Vergären (1) eine weitere Reinigung (10), insbesondere mittels Adsorption des Biogases durchgeführt wird.

10. Verfahren nach einem der Ansprüche 1 bis 9, **dadurch gekennzeichnet, daß** die im Kraftwerk (4) gewonnene, thermische Energie zur Erzeugung von Wärme und/oder Kälte herangezogen wird.

11. Vorrichtung zum Umwandeln und Verwerten von Nebenprodukten der milchverarbeitenden Industrie, insbesondere von Molke, Molkepermeaten, Milchpermeaten oder dgl., **gekennzeichnet durch**:
eine Einrichtung (1) zum Vergären der Nebenprodukte, insbesondere von Molke (2), **durch** anaerobe Behandlung zur Erzeugung von Biogas (3);
ein Kraftwerk (4) zum Erzeugen von elektrischer und/oder thermischer Energie (5, 6) aus dem beim Vergären gewonnenen Biogas; und
eine Einrichtung (11, 12, 16) zum Abtrennen von Inhaltsstoffen, insbesondere zum Abtrennen von Calcium, Magnesium und Phosphaten und/oder von Proteinen, aus den zu vergärenden Nebenprodukten.

12. Vorrichtung nach Anspruch 11, **dadurch gekennzeichnet, daß** eine Einrichtung (13, 14) zum Zudosieren von Wasser bzw. Abwasser und/oder einer Stickstoffquelle, insbesondere von Harnstoff, vorgesehen ist.

13. Vorrichtung nach Anspruch 11 oder 12, **dadurch gekennzeichnet, daß** eine Einrichtung (18) zum Erwärmen der zu vergärenden Nebenprodukte vorgesehen ist.

14. Vorrichtung nach einem der Ansprüche 11 bis 13, **dadurch gekennzeichnet, daß** eine Vorrichtung (10) zum Reinigen des Biogases, insbesondere eine Vorrichtung zum Entschwefeln des Biogases, zwischen der Einrichtung (1) zum Vergären und dem Kraftwerk (4) vorgesehen ist.

15. Vorrichtung nach einem der Ansprüche 11 bis 14, **dadurch gekennzeichnet, daß** zwischen der Einrichtung (1) zum Vergären der Nebenprodukte und dem Kraftwerk (4) eine Speichervorrichtung (15) zum Speichern von erzeugtem Biogas vorgesehen ist.

16. Vorrichtung nach einem der Ansprüche 11 bis 15, **dadurch gekennzeichnet, daß** der Einrichtung (1) zum Vergären der Nebenprodukte ein Puffer bzw. Vorratstank (19) und/oder eine Konditionierungseinrichtung (16) vorgeschaltet ist.
